# EUROPEAN PATENT APPLICATION

(11) **EP 1 541 566 A1**
(43) Date of publication of application: **15.06.2005**
(21) Application number: 03028154.7
(22) Date of filing: 08.12.2003
(51) Int. Cl.: C07D 295/22, C07B 63/00, C07C 45/27, C07C 51/16

(54) **Process for the separation of organic hydroxylamine and nitrosonium compounds and its use in the oxidation of hydroxy compounds**

(71) Applicant: SCA Hygiene Products AB, 405 03 Göteborg (SE)
(72) Inventor: Jetten, Jan, 3701 JL Zeist (NL); Besemer, Arie, 3958 CC Amerongen (NL)
(74) Representative: HOFFMANN EITLE

(57) **Abstract**

The present invention relates to a process for the separation of a protonated organic hydroxylamine compound and an organic nitrosonium compound from each other under acidic conditions, wherein a mixture of protonated organic hydroxylamine compound and organic nitrosonium compound is brought in contact with a hydrophobic resin to retain the organic hydroxylamine compound on the hydrophobic resin, as well as a process for the oxidation of a hydroxy compound in the presence of an organic nitroxy compound and optionally a primary oxidant, using this separation process.

## Description

The present invention relates to a process for the separation of organic hydroxylamine and nitrosonium compounds and a process for the oxidation of hydroxy compounds wherein the separated nitrosonium compound is used as oxidant. These processes allow the design of an oxidation process wherein various species can be recycled in an economically advantageous manner.

### Background art

Nitroxy-mediated oxidations are a useful tool to convert primary alcohols to aldehydes and/or carboxylic acids or secondary alcohols to ketones. Organic nitroxy compounds suitable for this purpose must be capable of forming stable radicals and therefore, as a rule, are sterically shielded by at least one bulky group having a quaternary carbon atom in α-position to the nitrogen atom. Especially TEMPO (2,2,6,6-tetramethylpiperidine-N-oxyl formula I in the scheme below) and its derivatives have attracted a lot of interest over the last years since they allow the selective introduction of aldehyde and/or carboxylic acids into alcohol substrates such as polysaccharides.

The reaction can be conducted in three ways. First, a nitrosonium ion (III) which is the actual active species can be generated catalytically in situ from a nitroxide in the presence of a primary oxidant, such as hypochlorous acid/hypochlorite as shown in the scheme below. Depending on the process conditions, the nitrosonium ion will oxidize the alcohol, i.e. the hydroxy compound to the corresponding aldehyde (as shown in the scheme) or carboxy compound while being reduced itself to the hydroxylamine. Equimolar amounts of the resulting hydroxylamine and nitrosonium ion will then recombine (synproportionate) under suitable reaction conditions to form the nitroxy compound. Then, the catalytic cycle newly starts.

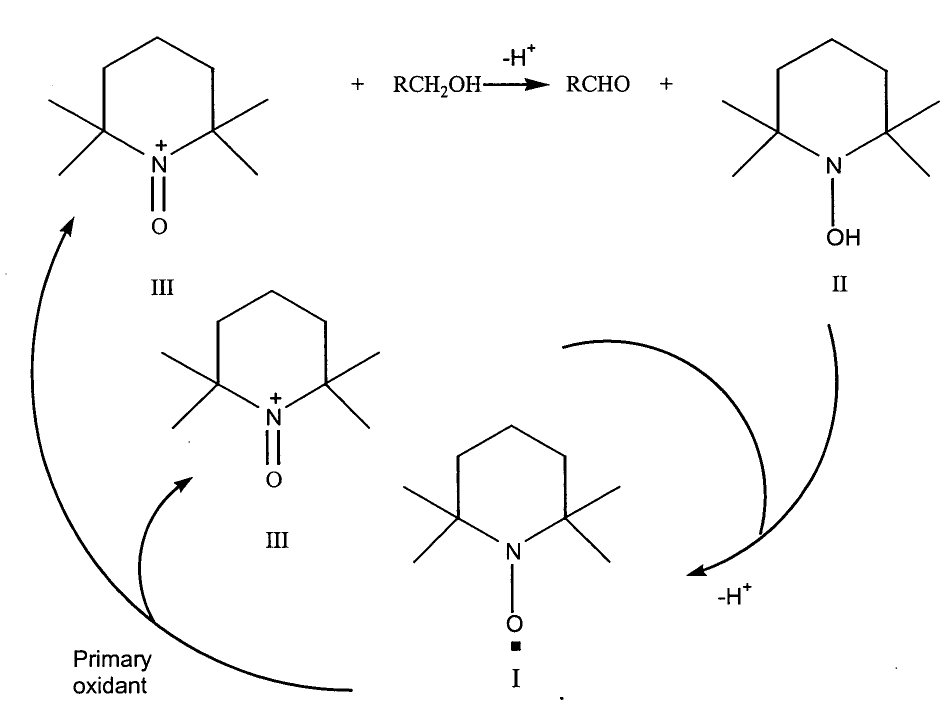

Second, the nitrosonium ion can be generated in situ by a disproportionation reaction of the nitroxy compound (I) to the corresponding hydroxylamine (II) and the nitrosonium ion (III) as shown in the following scheme. As the disproportionation reaction must be conducted under strongly acidic conditions, the hydroxylamine (II) is typically present in its protonated form (IV).

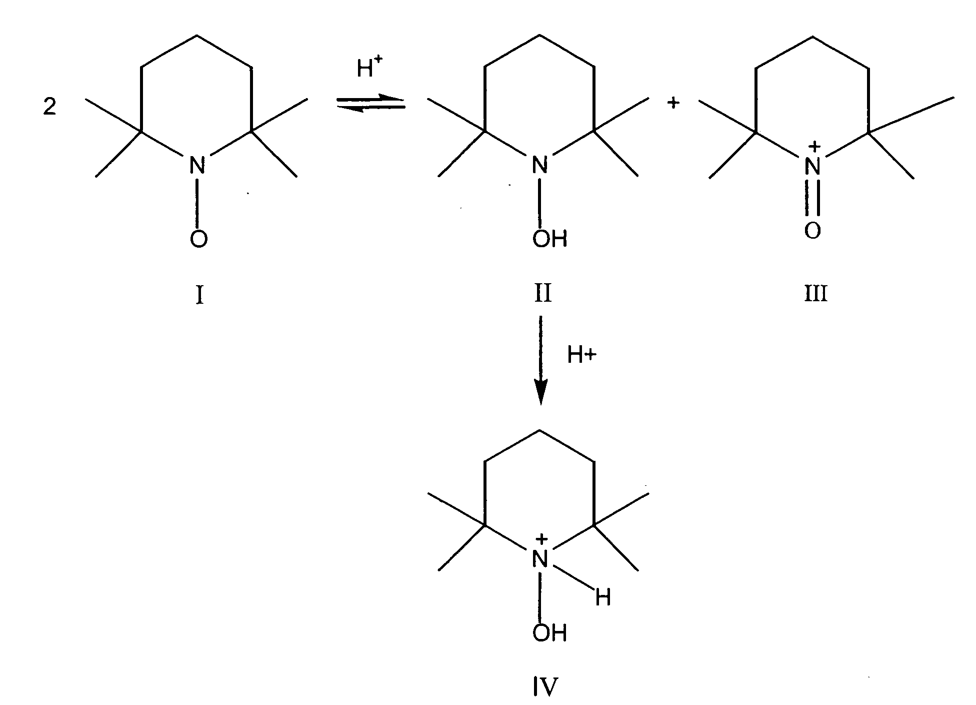

Third, it is also possible to prepare a salt of the nitrosonium ion, which then can be used in stoichiometric amounts.

Moreover, a relatively new technique involves the enzymatic conversion of nitroxy compounds as described in WO 00/50463.

There is extensive patent and scientific literature dealing with the synthesis and the use of nitroxy compounds, mostly TEMPO and its derivatives in oxidation reactions.

Reviews were published for instance by J.M. Bobbitt et al. in Heterocycles, vol. 27, No. 2, 1988 "Organic nitrosonium salts as oxidants in organic chemistry" and by A.E.J. De Nooy et al. in J. Synthetic Org. Chem 1996 (10),1153-1174 "on the use of stable organic nitroxyl radicals for the oxidation of primary and secondary alcohols".

If TEMPO and related nitroxy compounds are used as a catalyst in the oxidation of alcohols, for instance carbohydrates, the nitroxy compound is often lost during the isolation of the reaction product due to the relatively high volatility of nitroxy compounds such as TEMPO. This is an undesired situation for an economical process. Very few documents deal with technical solutions for overcoming this problem or the recovery of TEMPO in general.

WO 96/36621 proposes in this regard a method for obtaining a catalytically active mixture based on stable nitroxide radicals wherein at least a part of the reaction mixture is subjected to an azeotropic distillation. During the azeotropic distillation step the stable nitroxide radicals are at least partially distilled over with the liquid medium. However, this method requires distillation devices and creates high costs due to this energy consumption. Further, it cannot be applied to less volatile nitroxy compounds.

Another technique for avoiding the loss of TEMPO as a catalyst involves affixing the same to a polymer solid phase which then can be easily recovered from the reaction medium (T. Miyazawa, T. Endo et al. in J. Polymer Sci., Polymer Chem. Ed. 23 (1985) 1527-1535 and 2487-2494). Similarly, DE 42 09 869 A describes a method for immobilizing 4-hydroxy-TEMPO on polyvinyl-benzyl.

US 2003/0073871 pertains to the continuous oxidation of alcohol substrates to aldehydes in the presence of nitroxy compounds in an alkaline 2-phase system. The nitroxy compound can be present in the organic phase, in the aqueous phase or bound to solid supports.

However, the reaction rate of immobilized organic nitroxy compounds is typically lower than in homogeneous systems.

WO 02/59064 A1 relates to various recovery processes for TEMPO and related nitroxy compounds based on hydrophobic interactions. One option involves bringing the nitroxy compound into contact with hydrophobic resins such as XAD resins, for instance by passing the reaction mixture over a chromatographic column filled with one of these resins. The hydrophobic character of TEMPO and other organic nitroxy compounds leads to a strong affinity to hydrophobic resins thereby allowing the separation from the reaction mixture. Nevertheless, this recovery process creates new problems insofar as., after extracting the organic nitroxy compound from the column, the organic solvent (eluent) has to be removed by evaporation. At this point in time, the high volatility of some organic nitroxy compounds such as TEMPO or 3,4-dehydro-TEMPO leads again to an undesired loss of this costly material.

WO 02/58844 describes a special group of nitroxy compounds wherein 4 TEMPO molecules are integrated into a heterocyclic molecule and their use in the oxidation of alcohol substrates. These nitroxy compounds can be recovered by filtration or water evaporation steps. One method for the recovery of the nitroxy compound catalyst involves the filtration at pH values above 8. According to another embodiment, the organic phase containing the catalyst is stirred with concentrated hydrochloric acid (HCl) to transfer the catalyst into the aqueous phase followed by evaporating the water under formation of the catalyst salt.

Further, it is known in the art that N-methylmorpholine-N-oxide (NMMO) which is used as solvent in the Lyocell spinning process can be recovered and purified by means of anion exchange resins (H.Mertel et al., Papier (Darmstadt), 46 (3), 101-5, 1992, "Purification, recovery and methods for quantitative determination of N-methylmorpholine-N-oxide". The separation of amine oxides, such as NMMO from aqueous solutions by means of cationic exchange resins having carboxylate anchor groups is further known from EP 0 468 951 A1.

In view of the above, there is a strong need for an efficient recovery process for TEMPO, related organic nitroxy compounds and/or the catalytically active species thereof, i.e. the corresponding nitrosonium ion.

Furthermore, considering the sensitivity of many hydroxy compound substrates towards the primary oxidant, it would be desirable to design an oxidation process wherein the direct contact between hydroxy compound substrate and primary oxidant can be avoided and only the active species, the nitrosonium ion is included in the reaction mixture.

Therefore, it is one object of the present invention to provide a process for the separation of organic hydroxylamine and organic nitrosonium ion compounds, since the latter can be employed as active oxidizing species in nitroxy-mediated reactions.

It is a further object of the present invention to provide a separation process for organic hydroxylamine and organic nitrosonium ion compounds that makes an oxidation process possible wherein only the nitrosonium ion, but no primary oxidant is contacted with the hydroxy compound substrate.

It is a further object of the present invention to provide a process for the oxidation of hydroxy compounds wherein the separation of hydroxylamine and nitrosonium ion is used for recycling the actual oxidising species, i.e. the nitrosonium ion to the reaction mixture.

Moreover, it is an object of the present invention to provide a process for the oxidation of a hydroxy compound wherein the volatility of TEMPO and some related nitroxy compounds does not pose any problems.

Finally, it is an object of the present invention to provide a process for the oxidation of hydroxy compounds facilitating the recycling of various starting materials including the actual oxidising species (the nitrosonium ion) and the purification of the products formed during oxidation.

### Brief description of the invention

The above technical objects are solved by the following processes :
1. Process for the separation of a protonated organic hydroxylamine compound and an organic nitrosonium compound from each other under acidic conditions, said process comprising the steps of
   (i) bringing the mixture of protonated organic hydroxylamine compound and organic nitrosonium compound in contact with a hydrophobic resin to retain the organic hydroxylamine compound on the hydrophobic resin,
   (ii) separating the nitrosonium ion compound from the organic hydroxylamine compound retained on the hydrophobic resin.
2. Process for the oxidation of a hydroxy compound in the presence of an organic nitroxy compound and optionally a primary oxidant, comprising the steps of
   (a) separating an organic nitrosonium compound from a mixture comprising an organic nitrosonium compound and an organic hydroxylamine compound in accordance with the process defined above,
   (b) reacting the nitrosonium compound separated according to step (a) with a hydroxy compound to oxidize said hydroxy compound.

### Detailed description of the invention

According to the **first step** of the claimed **separation process**, an acidic mixture containing the organic hydroxylamine and nitrosonium ion compounds is contacted with a hydrophobic resin. Preferably, this mixture is passed over a column containing a hydrophobic resin.

"Acidic conditions" preferably means a pH value of less than 4, more preferably less than 3, even more preferably less than 2 (throughout the specification and claims, the pH values refer to a measurement of the respective system at 20°C).

Surprisingly, it has been found that the organic hydroxylamine, though being typically protonated under acidic conditions, is retained on the hydrophobic column, while the nitrosonium ion can be eluted.

The mixture to be separated preferably contains the organic hydroxylamine and nitrosonium compound in dissolved form.

The solvent is preferably water, or a mixture of water and a water-miscible organic solvent.

If water is used as medium, the organic hydroxylamine compound will always show a sufficient affinity to the hydrophobic resin in the following separation step due to strong interactions between the resin surface and hydrophobic molecule parts of the hydroxylamine compound. Regarding mixtures of water and a water-miscible organic solvent, the content of organic solvent should not exceed limits reducing or preventing the retention of the hydroxylamine compound on the hydrophobic resin in the subsequent step. This depends primarily on the type of hydroxylamine compound, resin and organic solvent. In this respect, a skilled person is capable to select suitable conditions including an appropriate ratio of water/organic solvent. Preferred volume ratios of water/organic solvent are in the order of more than 50%, in particular at least 80% water.

Furthermore, the use of relatively low-boiling water-miscible organic solvents having a boiling point of less than 150°C (at ambient pressure of 1013mbar), preferably less than 120°C, in particular less than 100°C is preferred.

Examples of the water-miscible organic solvents are water-miscible alcohols (e.g. ethanol, 1- or 2-propanol, t-butanol), ethers, ketones or nitriles, preferably non-alcoholic water-miscible solvents such as ethers (e.g. tetrahydrofuran (THF), dioxane), ketones (e.g. acetone) or nitriles (e.g. CH₃CN). The term "water-miscible" is to be understood as complete (concentration-independent) mutual solubility at 20°C.

Hydroxylamine and nitrosonium compound preferably comprise the same organic residue, i.e. they differ only in the presence of a hydroxylamine and nitrosonium functionality, respectively. Mixtures containing equimolar amounts of organic hydroxylamine and nitrosonium ion can be prepared by subjecting organic nitroxy compounds to disproportionation reaction under acidic conditions (pH below 4, preferably below 3, in particular below 2).

The organic nitroxy compound is preferably a sterically hindered organic nitroxy compound. (This, as well as the following description of structural features, also naturally applies to the corresponding nitrosonium and hydroxylamine compounds). One, particularly two bulky groups in the α position to the NO is/are suitable for sterical hindrance of the NO group, e.g. optionally substituted phenyl or aliphatic substituents that are linked to the nitrogen atom of the NO by a quaternary C atom, e.g. tert-butyl. In other words, it is preferred that one, in particular two quaternary carbon atoms are present in α-position of the N-atom. According to preferred embodiments, one, in particular both α-carbon atoms are dimethylsubstituted. It is similarly preferred that the nitroxy compound lacks α-hydrogen atoms. Two substituents can also be combined into an alkylen unit optionally interrupted by a hetero-atom (e.g. O,N) (to form an alicyclic or heterocyclic ring). The molecular weight of the nitroxy compound is preferably less than 500, its carbon number preferably less than 40, in particular less than 30, e.g. less than 20. It is also preferred that the nitrogen atom of the NO group is linked to only two organic residues (secondary nitroxy) and not three as for instance in NMMO (N-methyl morpholin-N-oxide).

Preferred nitroxy compounds can be represented by the following formula (V) 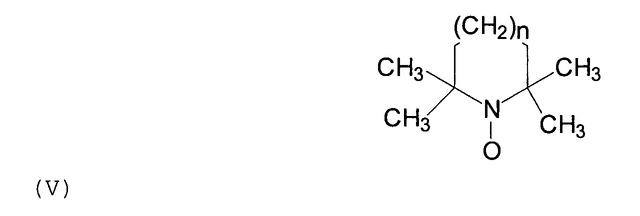 where n = 0 or 1 and where the methylene groups of the ring may carry one or more substituents selected from alkyl, alkoxy, aryl, aryloxy, amino, amido (e.g. acetamido, 2-bromacetamido and 2-iodacetamido), oxo, cyano, hydroxy, carboxyl, phosphonooxy, maleimido, isothiocyanato, alkyloxy, fluorophosphinyloxy (particularly ethoxyfluorophosphinyloxy), substituted or unsubstituted benzoyloxy, e.g. 4-nitrobenzoyloxy. If n = 1 (i.e. the ring represents a piperidine), these groups typically substitute the 4-position of the piperidine. Examples are 4-acetamido-TEMPO, 4-acetoxy-TEMPO or 4-hydroxy-TEMPO. The di-tert.-alkyl nitroxy unit can also be present as part of a polymer structure such as {(CH₃)₂-C-(CH₂)₂₋₃-(CH₃)₂-C-NO-}ₘ- . Hydroxy, amino and amido are preferred among these substituents on account of the stability of the nitroxy compound under acidic conditions. The ring may also be partially unsaturated as in 3,4-dehydro-TEMPO.

An example of n=0 is PROXYL (2,2,5,5-tetramethylpyrrolidine-N-oxyl).

Among the formula (V) compounds, the case of n=1 is preferred. This leads to the optionally substituted TEMPO compounds (2,2,6,6-tetramethyl-4-piperidine-N-oxide) which can selectively oxidize the primary hydroxy group at C(6) of the glucose unit of the cellulose into aldehyde and/or carboxyl groups.

DOXYL (4,4-dimethyloxazolidine-N-oxyl) can also be used as nitroxy compound in the present invention.

As to the disproportionation reaction, any acid can be used, e.g. perchloric acid, although it is generally preferred to employ non-oxidising organic or inorganic acids for adjusting the pH to the aforementioned acid values. In order to reach such low pH values it is generally advantageous to use stronger acids, for instance those having pK values (at 25°C in an aqueous solution) of less than 5, preferably less than 3, for instance less than 1 in particular less than -1. Very strong acids fulfilling even the latter requirement are sulfuric acid or hydrochloric acid.

If the entire recovery and oxidation process is to be conducted under TCF (total chlorine-free) conditions, it is preferred to use chlorine-free acids, such as organic sulfonic acids (e.g.benzenesulfonic acid, o- or p-toluenesulfonic acid), sulfuric acid, nitric acid or phosphoric acid.

To achieve disproportionation, the organic nitroxy compound is preferably dissolved in a suitable solvent and then treated with an acid. The solvent is preferably the same as used for the subsequent (chromatographic) separation by means of a hydrophobic resin. It is preferred to use polar solvents, in particular water, water-miscible organic solvents and mixtures thereof. Examples for the water-miscible organic solvents were already mentioned. Non-alcoholic solvents such as tetahydrofuran (THF), dioxane, acetone or CH₃CN are preferred.

Suitable resins can be selected from non-ionic, hydrophobic, crosslinked resins. These resins preferably contain, as major monomeric unit in terms of molar amount, polymerizable aromatic monomeric units such as styrene or divinylbenzene or vinyl monomers such as (meth)acrylic acid. Crosslinking may take place during and/or after polymerisation. These resins preferably display a macroreticular structure involving both a continuous polymer phase and a continuous pore phase. The harmonic mean size of the resin beads preferably ranges from 0,4 to 0,7 mm. Furthermore, it is preferred if the surface area is above 380 m²/g and/or the porosity above 0,5 ml/ml.

Such resins are available under the trade name XAD® from Sigma, USA, Supelco, Bellefonte, PA, USA or Rohm & Haas company, USA. Suitable types are for instance XAD-2, XAD-4, XAD-8, XAD-11, XAD-16, XAD-30, or XAD-1180.

There are no specific limitations regarding the concentration of hydroxylamine and nitrosonium ion as long these are soluble in the solvent chosen. Their total concentration preferably ranges from 0,1 to 50 weight%, based on the pure solvent (mixture).

The separation step is preferably conducted at ambient temperature (20 to 25°C), although temperatures in the order of, for instance, 0 to 50°C are also applicable.

The **second step** of the claimed separation process involves separating the nitrosonium ion compound from the hydroxylamine compound retained on (adsorbed on) the hydrophobic resin.

This can be achieved by filtrating the hydrophobic resin from the solvent containing the nitrosonium ion compound followed by an optional washing step for the hydrophobic resin.

In the preferred case of chromatographic separations, the column containing the hydrophobic resin is eluted with a suitable solvent until fractions containing the nitrosonium ion compound can be collected. The washing/elution steps preferably employ the same solvent as described above, i.e. water or a mixture of water and the water-miscible organic solvent.

As regards the desorption of the hydroxylamine still retained on the hydrophobic resin, the following is to be observed.

The hydroxylamine compound is preferably removed from the hydrophobic resin by elution with a water-miscible organic solvent or a suitable mixture of water and a water-miscible organic solvent. The water-miscible organic solvent is preferably selected among the already described ones. At any rate, it is necessary that the eluent is less polar (more hydrophobic) than the mixture from which the hydroxylamine compound was adsorbed. Thus, organic solvents such as ethanol, 1- or 2-propanol, t- butanol, acetone, THF, dioxane, CH₃CN, or their mixtures with water, are capable of eluting hydroxylamine compounds that were retained from an aqueous mixture.

Similarly, a mixture of water and water-miscible organic solvent having a higher content of organic solvent (per volume ratio, e.g. at least 5% or 10% more) is basically suitable to elute organic hydroxylamine compounds that were retained from a mixture of water and organic solvent having a lower content of organic solvent. A suitable difference in terms of volume ratio can be easily determined by a skilled person.

Because of the incompatibility of some organic solvents with oxidants used in the oxidation process described below, it is often beneficial to remove organic solvents if present in the eluate.

The present invention also relates to a **process for the oxidation of a hydroxy compound,** comprising the steps of
a) separating an organic nitrosonium compound from an organic hydroxylamine compound in accordance with the previously described separation process, followed by
b) reacting the nitrosonium compound separated from the hydroxylamine with a hydroxy compound, e.g. by feeding the nitrosonium compound to a reaction vessel containing the hydroxy compound (in a suitable medium) to oxidize said hydroxy compound.

According to one embodiment of this process, it is preferred to generate the mixture comprising an organic nitrosonium compound and an organic hydroxylamine by disproportionating an organic nitroxy compound, as described before, with an acid. This preferred step is conducted prior to step (a).
Should the organic nitrosonium compound after separation be dissolved or dispersed in a medium containing or consisting of the water-miscible organic solvent, it is preferred to remove this organic solvent if the same is susceptible to undesired oxidation reactions in the following steps.

According to the present invention, there are no specific limitations regarding the hydroxy compound to be oxidized in **step (b)**. It is one feature of nitroxy-mediated oxidations that they are compatible with a huge variety of different substrate hydroxy compounds. Thus, the hydroxy compound can comprise either primary or secondary hydroxy functionality. The oxidation of primary hydroxy compounds is preferred since techniques known in the art allow conducting the nitroxy-mediated oxidation of primary hydroxy compounds with particularly high selectivity. The primary hydroxy compound is oxidized to the corresponding aldehyde and/or carboxylic acid. The interruption of the oxidation process and/or a suitable reaction conditions enable the isolation of the intermediate aldehyde. Interrupting the oxidation process is particularly suited for the oxidation of polysaccharides.

Examples for suitable hydroxy compound substrates are low molecular weight (up to MW 1000) aliphatic or aromatic-aliphatic hydroxy compounds such as oligosaccharides, as well as hydroxy compounds having a higher molecular weight including polymeric hydroxy compounds such as polysaccharides. The oxidation of oligo- and polysaccharides, such as starch or cellulose typically leads to the corresponding C6-aldehyde and/or carboxy derivates. The oxidation of cellulose and cellulose- containing materials, such as pulp is particularly preferred.

The starting pulps which may be used for oxidation may relate to primary fibrous materials (raw pulps) or to secondary fibrous materials, whereby a secondary fibrous material is defined as a fibrous raw material recovered from a recycling process. The primary fibrous materials may relate both to a chemically digested pulp (e.g. Kraft or sulfite pulp) and to mechanical pulp such as thermorefiner mechanical pulp (TMP), chemothermorefiner mechanical pulp (CTMP) or high temperature chemithermomechanical pulp (HTCTMP). Synthetic cellulose-containing fibers can also be used. Preference is nevertheless given to the use of pulp from plant material, particularly wood-forming plants. Fibers of softwood (usually originating from conifers), hardwood (usually originating from deciduous trees) or from cotton linters can be used for example. Fibers from esparto (alfa) grass, bagasse (cereal straw, rice straw, bamboo, hemp), kemp fibers, flax and other woody and cellulosic fiber sources can also be used as raw materials. The corresponding fiber source is chosen in accordance with the desired properties of the end product in a manner known in the art.

The oxidized pulps can be used for the manufacture of paper, in particular tissue paper having improved strength properties. Prior to or after oxidation the starting pulps can be beaten (refined) with the aim of further enhancing paper strength. In the manufacture of oxidized pulps it is further preferred to carry out all oxidation steps in the absence of chlorine-containing oxidants as basis for the production of TCF or ECF paper.

The hydroxy compound to be oxidized is dissolved or dispersed in a suitable reaction medium, for instance water, the already-mentioned water-miscible (preferably non-alcoholic) organic solvents such as THF, dioxane, acetone or CH₃CN or their mixtures with each other or water.

As to step (b) of the claimed oxidation process, the nitrosonium compound is preferably used in molar amounts of 0.04 to 4.4 mol with respect to the hydroxy functionality to be oxidized. This also includes the possibility of partially oxidizing polyhydroxy compounds such as cellulose present in pulp.

A list of suitable reaction conditions is found in A.E.J. De Nooy, Synthesis 1996, 1153-1174. Once the nitrosonium compound has been obtained in a relatively pure form (no or little hydroxylamine content), the oxidation can be carried out in a relatively wide pH range of preferably 1 to 11,5.

Other preferred reaction conditions are as follows:
- a reaction temperature of 0 to 50°C
- a concentration of the hydroxy compound in the aqueous or organic solvent from 0.1 to 50 wt%, in particular 0.5 to 10 wt%, based on the weight of the solvent,
- a molar ratio hydroxy functionality/nitrosonium ion of 0.04 to 4.4 mol.

According to preferred **step (c)**, a suitable oxidizing agent is reacted with the hydroxylamine retained on the hydrophobic resin to form the corresponding nitrosonium ion, which then can be eluted according to **step (d)**. For the elution step (d) preferably the same solvents (water, water-miscible organic solvent or mixture thereof) are used as already described above, in the context of the separation process of the invention.

These steps can be performed by passing a suitable oxidizing agent over the column containing the hydrophobic resin on which the hydroxylamine is adsorbed.

When selecting a "suitable" oxidizing agent among those known in the art and/or specifically mentioned in the present specification it is to be taken into account that, depending on the type of hydrophobic resin used, the oxidative degradation of this resin is to be minimized. Good results have already been achieved with OCl⁻/HOCl.

The oxidation step (c) (similarly as step (d') explained below) is preferably performed under acidic condition. pH values of below 4, more preferably below 3, and even more preferably below 2 can be used. These acidic condition will ensure that the synproportionation reaction between (not yet oxidized) hydroxylamine compound and nitrosonium ion compound, as generated during the oxidation, is suppressed.

Suitable oxidants can be selected among primary oxidants typically used together with organic nitroxy compounds such as chlorine, bromine, iodine, hypochlorite, chlorite (in combination with hypochlorite), hypobromite, iodite, Fe(CN)₆³⁻, transition metals of periods Va to VIIIa in the oxidation state of at least +3, oxidases, ozone, hydrogen peroxide, peroxosulfate and/or peracids. For each of these primary oxidants suitable reaction conditions, as known in the art, are to be selected. Thus, it can for instance be undesired to use hypochlorite at pH <2 since then chlorine is formed, and tends to escape form the reaction mixture.

It is preferred to use this oxidizing agent in approximately stoichiometric amounts, preferably stoichiometric amounts, based on the molar amount of hydroxylamine adsorbed on the hydrophobic resin. "Approximately stoichiometric" means a stoichiometric amount ± 20%, preferably ± 10% of the stoichiometrically required molar amount. This ensures that only small amounts of the oxidant remain in the mixture eluted from the hydrophobic resin.

Alternatively, the oxidant is added in a molar excess with respect to the stoichiometrically required amount, for instance in amounts of more than 1,2 mol, more preferably more than 1,5 mol, even more preferably more than 2 mol (e.g. up to 5 mol), if the stoichiometrically required amount is assumed to be 1. Then, it is preferred to reduce this excess prior to any recycling steps. An excess of sodium hypochlorite can for instance be reduced by hydrogen peroxide. This leads to the formation of sodium chloride which is tolerable for a chlorine-free process.

One version of performing the oxidation according to step (c) lies in the oxidation of the hydroxylamine with a peracid, a precursor or a salt thereof as a primary oxidizing agent, in the presence of a catalytic amount of a halide (e.g. NaBr), preferably in the pH range of 2 to 4, particularly 2.5 to 3.5, similarly as disclosed in WO 99/57158. The peracid is preferably a peralkanoic acid, particularly peracetic acid, or persulfuric acid. It is, however, also possible to perform oxidation just using peracid (e.g. persulfuric acid) as an oxidizing agent without halide.

Another version lies in the reaction between hydroxylamine and a suitable oxidic compound of a metal of the transition metals of periods Va to VIIIa in the oxidation state of at least +3, e.g. oxides and oxygen-containing ions of manganese, chromium, iron, nickel, ruthenium and vanadium, e.g. vanadium pentoxide, iron oxide, chromium (VI) oxide, chromates and particularly manganese (IV) oxide and salts of permanganic acid. The reaction is preferably conducted at a pH between 2 and 4. The reaction temperature is preferably less than 80°C, particularly 30 to 60°C. This technique is similar to the description of WO 01/00681.

Another version lies in the oxidation of hydroxylamine compounds, preferably those derived from 4-hydroxy-, 4-amino- or 4-amido-substituted TEMPO) at a pH between 1 and 4, particularly 2 to 3. In this version, a hypohalite (e.g. NaOCl) or ozone is particularly suitable as a primary oxidizing agent. The reaction temperature is preferably 5 to 50°C. Halogen-free acids, such as sulfuric acid or toluenesulfonic acid, are particularly suitable for setting the pH.

According to an alternative and preferred embodiment over steps (c) and (d) (in the following referred to as **steps (c') and (d')**), a first solvent (eluent), such as water or a mixture of water and a water-miscible organic solvent (e.g. the already mentioned ones) is used in step (a) for retaining the hydroxylamine on the hydrophobic resin and eluting the nitrosonium ion, preferably from a column containing said hydrophobic resin. According to this alternative embodiment, after step (b), a second less polar solvent (eluent) is used for eluting the retained hydroxylamine from the hydrophobic resin **(step c')**, preferably from a column containing the same. The hydroxylamine obtained thereby is oxidized to the nitrosonium ion **(step d')**.

This embodiment avoids potential problems which may occur if, in line with steps (c) and (d), an oxidant is contacted with the hydrophobic resin, for instance due to an undesired oxidative degradation of the hydrophobic resin.

The above-mentioned second solvent (eluent) can be a mixture of water and a water-miscible organic solvent having a lower content of water than the first solvent (eluent) eluent. It may also consist of a water-miscible organic solvent, such as acetone, which is preferred. The use of a second less polar (more hydrophobic) solvent (eluent) reduces the strong hydrophobic interactions between the hydroxylamine retained on the column and the hydrophobic resin thereby causing its elution. As explained it can be preferred to remove the organic solvent prior to the oxidation steps.

According to step (d'), the eluted hydroxylamine is then oxidized to the corresponding nitrosonium ion, for instance with the same primary oxidants and under the same conditions as already described for step (c) and (d). For the reasons given above, step (d') is preferably performed under acidic conditions.

According to step (d'), the hydroxylamine can also be oxidized with enzymes and/or metal complexes, enzymes being preferably combined with a small amount of organic nitroxy compound. The enzymes to be used according to this embodiment are oxidoreductases or other enzymes that are capable of oxidation in the presence of a suitable redox system. Oxido-reductases, i.e. enzymes capable of oxidation without the presence of further redox systems, to be used include peroxidases and oxidases, in particular polyphenol oxidases and laccase. Certain hydrolases, such as phytase, can be used when a further redox system is present such as a metal complex, e.g. vanadate. Metal complexes as such, without an enzyme protein, can also be used; examples include copper and iron complexes with porphyrins, phenanthrolins, polyamines such as EDTA, EGTA and the like. The metal-assisted enzymes and metal complexes require hydrogen peroxide, alkyl and ar(alk)yl hydroperoxides (such as tert-butyl hydroperoxide) or chlorite as an ultimate electron acceptor. Peroxidases (EC 1.11.1.1 - 1.11.1.11) that can be used according to the invention include the peroxidases which are cofactor-independent, in particular the classical peroxidases (EC 1.11.1.7). Peroxidases can be derived from any source, including plants, bacteria, filamentous and other fungi and yeasts. Examples are horseradish peroxidase, soy-hull peroxidase, myelo peroxidase, lactoperoxidase, Arthromyces and Coprinus peroxidases. Several peroxidases are commercially available. The peroxidases require hydrogen peroxide as an electron acceptor. Polyphenol oxidases (EC 1.10.3.1.) include tyrosinases and catechol oxidases such as lignine peroxidase. Suitable polyphenol oxidases may be obtained from fungi, plants or animals. The polyphenol oxidases require oxygen as an electron acceptor. Laccases (EC 1.10.3.2) are sometimes grouped under the polyphenol oxidases, but they can also be classified as a distinct group, sometimes referred to as p-diphenol oxidases. The laccases can be derived from plant sources or from microbial, especially fungal, sources. The laccases also require oxygen as an electron acceptor. The process for producing the nitrosonium ion according to this embodiment can be performed under relatively mild conditions, e.g. at a pH between 2 and 4, and at a temperature between 15 and 60°C (both depending on the particular enzyme or metal complex). The reaction medium can be an aqueous medium.

According to a preferred embodiment of the claimed oxidation process, the nitrosonium compound obtained in step (d) or (d') is reacted with a hydroxy compound, e.g. by feeding the nitrosonium compund to a reaction vessel containing a hydroxy compound, thereby oxidising said hydroxy compound.

Thus, this embodiment is advantageous insofar as the active species, i.e. the nitrosonium ion is generated separately from the reaction mixture. In this manner, undesired side reactions occurring between the hydroxy compound substrate and the primary oxidant can be avoided. Further, the nitroxy compound can be efficiently utilised since, after disproportionation, not only the oxidized species (nitrosonium ion), but also the reduced form (hydroxylamine), after its oxidation to the nitrosonium ion, is included in the reaction mixture.

Another feature makes the oxidation process of the present invention particularly attractive. All oxidation and separation steps can be conducted in the presence of the relatively polar nitrosonium ion and hydroxylamine which is typically protonated under strongly acidic (disproportionation) conditions. Thus, in contrast to oxidation cycles of the prior art where the corresponding nitroxy compound per se, such as TEMPO is present, the volatility of the oxidant does not pose any problems.

The inventor's discovery that hydroxylamine and nitrosonium ion separate on hydrophobic columns can also be used for purifying the reaction mixture. This reaction mixture which may contain, apart from the mixture of unreacted nitroxy compound and its reduced (consumed) form, i.e. the hydroxylamine, unreacted hydroxy compound and/or its oxidation products and/or nitroxy compound is passed over the column containing a hydrophobic resin in order to separate unreacted hydroxy compound and/or oxidation products and/or the organic nitrosonium compound from the reaction mixture while the nitroxy compound/or the hydroxylamine are retained.

In line with this embodiment, it is preferred to isolate the oxidation products from the eluent while feeding unreacted hydroxy compound and/or the nitrosonium ion back to the reaction vessel where the oxidation occurs.

The invention will now be illustrated by the following example.

### Example 1

250 ml of an aqueous solution containing 434 mg TEMPO (2,8 mmol) was disproportionated with 5 ml of hydrochloric acid (37%) for 2 hours at 50°C. The solution was eluted over a glass column containing 20g of a hydrophobic resin (XAD 1180 available from Supelco, Belfonte, US). The absorbance of the eluate at 430 nm (TEMPO) and at 480 nm (nitrosonium ion = TEMPO⁺) was measured . Next the pH of one part of the eluate was adjusted to 7 by addition of sodium carbonate and the absorbance measurements at 430 and 480 nm were repeated. To the neutralised (pH 7) eluate some α-methyl glucopyranoside (+α MGP) was added, after which the absorbance at 430 nm and 480 nm was measured again. The results are shown in Table 1.

**Table 1**

| Sample type | E at λ=430 nm (TEMPO) | E at λ=480 nm (TEMPO⁺) |
|---|---|---|
| Original eluate | 0,065 | 0,100 |
| Eluate pH7 | 0,067 | 0,103 |
| Eluate pH7+α MGP | 0,106 | 0,067 |

From the data expressed in Table 1, it can be concluded that synproportionation is not occurring due to the absence of the corresponding hydroxylamine. Hence the absorbance at 430 nm and 480 nm after neutralization (pH7) of the eluate do not significantly differ from those of the original eluate. Only after addition of a suitable oxidation substrate, e.g. α-methyl glucopyranoside, a clear shift in absorbance towards 430 nm was observed.

Incidentally, the hydroxylamine remaining on the column could be eluted with acetone.

### Example 2

One part of the eluate as obtained in Example 1 (115 ml, containing 0.64 mmol of nitrosonium ion) was neutralized to pH 9 by addition of sodium carbonate. To this solution 24.7 mg α-methyl glucopyranoside (0.127 mmol) was added. The pH was kept constant at 9 by addition of sodium carbonate. After 8 hours the amount of uronic acid was measured according to the Blumenkrantz method. The α-methyl glucopyranoside was oxidized into the corresponding uronic acid to a measured degree of 102.1 ± 6%. (It is to be noted that for a stoichiometric conversion of 1 mol MGP, 4 mol TEMPO⁺ is needed).

## Claims

1. Process for the separation of a protonated organic hydroxylamine compound and an organic nitrosonium compound from each other under acidic conditions, said process comprising the steps of
(i) bringing the mixture of protonated organic hydroxylamine compound and organic nitrosonium compound in contact with a hydrophobic resin to retain the organic hydroxylamine compound on the hydrophobic resin,
(ii) separating the nitrosonium ion compound from the organic hydroxylamine compound retained on the hydrophobic resin.

2. Process according to claim 1 wherein
(i) the mixture of organic hydroxylamine and organic nitrosonium ion compounds is passed over a column containing a hydrophobic resin, and
(ii) the nitrosonium ion compound is eluted while the organic hydroxylamine compound is retained on the column.

3. Process according to claim 1 or 2 wherein the mixture of organic hydroxylamine and organic nitrosonium ion compounds is generated by disproportionating an organic nitroxy compound at a pH below 4.

4. Process according to claim 3, wherein the nitroxy compound has the following formula (V): 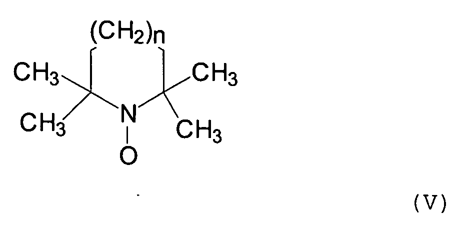 where n = 0 or 1 and where the methylene groups of the ring may carry one or more substituents selected from alkyl, alkoxy, aryl, aryloxy, amino, amido, oxo, cyano, hydroxy, carboxyl, phosphonooxy, maleimido, isothiocyanato, alkyloxy, fluorophosphinyloxy, substituted or unsubstituted benzoyloxy.

5. Process according to any of claims 1 to 4 wherein the organic hydroxylamine compound and organic nitrosonium compound to be separated from each other are present in dissolved form in water, or a mixture of water and water-miscible organic solvent.

6. Process according to any of claims 1 to 4 wherein the organic nitrosonium compound is separated in step (ii) by washing or eluting the hydrophobic resin with water or a mixture of water and water-miscible organic solvent.

7. Process according to any of claims 1 to 4 wherein the process comprises a further step of desorbing the hydroxylamine retained on the hydrophobic resin with a water-miscible organic solvent or a mixture of water and a water-miscible organic solvent.

8. Process according to claim 5 to 7 wherein the water-miscible organic solvent is selected from water-miscible alcohols, ethers, ketones or nitriles.

9. Process according to any of claims 1 to 8 wherein the hydrophobic resin is selected from crosslinked styrene-based or (meth)acrylic acid-based resins.

10. Process for the oxidation of a hydroxy compound, comprising the steps of
(a) separating an organic nitrosonium compound from a mixture comprising an organic nitrosonium compound and an organic hydroxylamine compound in accordance with the process defined in any of claims 1 to 9,
(b) reacting the nitrosonium compound separated according to step (a) with a hydroxy compound to oxidize said hydroxy compound.

11. Process according to claim 10 wherein the mixture subjected to the separation step (a) is obtained by treating an organic nitroxy compound with an acid to disproportionate the same to the corresponding nitrosonium and hydroxylamine compounds.

12. Process according to claim 9, wherein the mixture subjected to the separation step (a) is the reaction mixture obtained from the oxidation of a hydroxy compound which, apart from nitrosonium and hydroxylamine compounds, may also contain unreacted hydroxy compound and/or its oxidation products and/or organic nitroxy compound, and this reaction mixture is passed over a column containing a hydrophobic resin in order to separate the unreacted hydroxy compound and/or its oxidation products and/or the organic nitrosonium compound from the reaction mixture while the nitroxy compound and/or the hydroxylamine are retained.

13. Process according to claims 10, 11 or 12 wherein, after step (b),
(c) a suitable oxidizing agent is reacted with the hydroxylamine compound retained on the hydrophobic resin to form the corresponding nitrosonium ion compound on the hydrophobic resin, and
(d) the nitrosonium ion compound produced thereby is eluted from the hydrophobic resin.

14. Process according to claim 13 wherein the oxidizing agent is passed over a column containing the hydrophobic resin, and the nitrosonium ion compound is eluted from this column.

15. Process according to claim 10, 11 or 12 wherein in step (a) a first solvent, such as water is used for retaining the hydroxylamine on the hydrophobic resin and eluting the nitrosonium ion and, after step (b),
(c') a different less polar water-miscible organic solvent, such as acetone is used for eluting the retained hydroxylamine from the hydrophobic resin, optionally this organic solvent is removed, and
(d') the hydroxylamine obtained thereby is oxidized to the nitrosonium ion.

16. Process according to claim 13 or 15 wherein, after step (d) or (d'),
(e) the nitrosonium ion obtained is fed to said reaction vessel containing a hydroxy compound thereby oxidizing said hydroxy compound.

17. Process according to claim 12 wherein the oxidation products are isolated and the unreacted hydroxy compound and/or the nitrosonium ion are fed back to the reaction vessel.

18. Process according to any of claims 10 to 17 wherein the hydroxy compound is cellulose present in pulp.

19. Process according to claim 18 wherein the oxidation is carried out in the absence of chlorine-containing oxidants.
